# EUROPEAN PATENT APPLICATION

(11) **EP 1 813 215 A1**
(43) Date of publication of application: **01.08.2007**
(21) Application number: 07101361.9
(22) Date of filing: 29.01.2007
(51) Int. Cl.: A61B 17/17

(54) **Tool**

(30) Priority: 30.01.2006 GB 0601803
(71) Applicant: Finsbury (Development) Limited, Leatherhead, Surrey KT22 0BA (GB)
(72) Inventor: Wozencroft, Robert Michael, Epsom, Surrey KT19 8SS (GB)
(74) Representative: Smaggasgale, Gillian Helen

(57) **Abstract**

An alignment guide for use in femoral head surgery comprising:
a support member comprising a cannulated rod;
at least three locator arms at least a portion of at least one of which comprises a cantilever spring, each of said arms having a proximal end connected to the support member and a distal end having location means for location at the femoral head/neck junction,
said at least three locator arms being spaced such that in use they extend around the femoral head and prevent relative movement between the alignment guide and the femoral head.

## Description

The present invention relates to a tool for use in hip resurfacing operations. More particularly, it relates to an alignment guide for assisting in the correct machining of the femoral head such that a replacement femoral head can be correctly situated.

The efficient functioning of the hip joints is extremely important to the well being and mobility of the human body. Each hip joint is comprised by the upper portion of the upper leg bone (femur) which terminates in an offset bony neck surmounted by a ball-headed portion which rotates within a socket, known as the acetabulum, in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular, or socket, component which lines the acetabulum; and a femoral, or stem, component which replaces the femoral head. During the surgical procedure for implanting the hip prosthesis the cartilage is removed from the acetabulum using a reamer such that it will fit the outer surface of the acetabular component of the hip prosthesis. The acetabular component can then be inserted into place. In some arrangements, the acetabular component may simply be held in place by a tight fit with the bone. However, in other arrangements, additional fixing means such as screws or bone cement may be used. The use of additional fixing means help to provide stability in the early stages after the prosthesis has been inserted. In some modem prosthesis, the acetabular component may be coated on its external surface with a bone growth promoting substance which will assist the bone to grow and thereby assist the holding of the acetabular component in place. The bone femoral head will be removed and the femur hollowed using reamers and rasps to accept the prosthesis. The stem portion will then be inserted into the femur.

In some cases, a femoral component of the kind described above may be replaced with components for use in femoral head resurfacing or for use in thrust plate technology.

Although the prosthesis being inserted when the head is being replaced or resurfaced or in thrust plate arrangements is relatively small, the requirement for the surgeon to obtain the necessary access to the hip joint means that it is necessary to make a large incision on one side of the hip. In one technique, a straight incision is made through the skin on the posterior edge of the greater trochanter. In some techniques this incision may be made when the hip is flexed to 45°. By known techniques, the muscles and tendons are parted and held by various retractors such that they do not interfere with the surgeon's access to the hip joint. The hip is then dislocated to provide access to the head of the femur.

It will be acknowledged that it is essential that the replacement surface for the head of the femur should be precisely located in both angular and translation positions of the axis of the femoral neck of the implant. To assist this, in some techniques, the surgeon inserts a pin in the lateral femur. The desired position of the pin will be known from pre-operative analysis of x-rays of the joint. The surgeon will measure the desired distance down the femur from the tip of the greater trochanter and the alignment pin is inserted through the vastus lateralis fibres. The alignment pin is inserted in a transverse direction into the mid-lateral cortex and directed upwardly towards the femoral head. The pin is left protruding so that an alignment guide can be hooked over the alignment pin. Suitable alignment guides include that known as the MclVlinn Alignment Guide available from Smith & Nephew Orthopaedics Limited.

The alignment guides of the kind described above generally comprise a hook or aperture which is placed over the alignment pin thus providing a good angular position for the axis of the implant in valgus, varus and ante-version of the neck. The guide will then be adjusted such that a cannulated rod is located with the aperture therein directed down the mid-lateral axis of the femoral neck. A stylus having been set to the desired femoral component size is positioned such that it can be passed around the femoral neck. When the stylus can be passed around the femoral neck, the cannulated rod is locked in position. Once the guide is stabilised in this way fine adjustments can be made until the surgeon is happy that the guide is in the required position.

A guide wire can then be inserted through the cannulated rod. This guide wire is then used in the further surgery in which the femoral head is shaped to accept the prosthesis. This shaping involves removing the top of the head at an appropriate position and then machining the sides of the head using a sleeve cutter. These sleeve cutters are arranged such that the diameter cut will be correct for the replacement head size chosen and will bottom on the top of the cut head such that the teeth of the cutter do not dangerously over-sail the head-neck junction and cause soft tissue damage or neck notching.

Thus the machining procedure usually comprises the steps of drilling a well into the head of the femur, removing the drill, removing the top of the head of the femur, inserting a guide rod into the well, locating a sleeve cutter on the guide rod and cutting the head and optionally chamfer cutting the head. However, it will be understood that the order of the steps may be altered.

An alignment guide is generally used to ensure that the aperture drilled in the femoral head is both central to the femoral neck and at the correct angle of alignment thereto and that the shaping of the femoral head is accurate for the chosen head size.

It will therefore be understood that it is very important that the alignment guide is positioned correctly. Failure to do so may have the disastrous effect of allowing the machining of the cylinder of the head during the shaping procedure to "notch" into the neck of the femur. This will predispose the bone to early failure on load bearing.

Various alignment guides have been proposed which allow the required incision in the hip to be as small as possible and the amount of interaction with healthy tissue to be minimised. This is achievable as these guides do not require the alignment pin required by previous devices to be inserted. Where these guides are used, all of the surgical procedure takes place at the femoral head and the positioning and angling of the guide wire is taken, by means of the alignment guides, from the femoral neck.

Examples of such alignment guides include EP1588668, EP1588669, US2005/0033290, US6595999, US5312409, US2005/0113841, DE10013331, US4896663, and US6156069. Whilst these alignment guides go some way to addressing the problems associated with the more invasive guides used historically, there is still a need for alternative arrangements which meet particular requirements. For example, it is desirable to provide an alignment guide which is simple to operate.

In addition, many of the known guides are located on the femoral neck itself. These are used in a similar manner to those described above and may involve some adjustment by the surgeon before he selects the best position.

Whilst many of these alignment guides provide satisfactory results, there is a need for alternatives for situations where the surgeon is unable or prefers not to use the femoral neck as the basis used by the guide to assess the correct angle. It is therefore desirable to provide alternative guides which utilise the underside of the femoral head as it connects to the femoral neck to provide the correct orientation for the alignment guide.

Thus according to the present invention there is provided an alignment guide for use in femoral head surgery comprising:
a support member comprising a cannulated rod;
at least three locator arms at least a portion of at least one of which comprises a cantilever spring, each of said arms having a proximal end connected to the support member and a distal end having location means for location at the femoral head/neck junction,
said at least three locator arms being spaced such that in use they extend around the femoral head and prevent relative movement between the alignment guide and the femoral head.

In one arrangement each locator arm may comprise a cantilever spring.

In a preferred arrangement, the alignment guide includes four locator arms at least a portion of each of which preferably comprises a cantilever spring. The use of four cantilever springs serves to centre the alignment guide on the femoral neck.

The use of the or each cantilever springs of the present invention enables any locator arm having the cantilever spring to move resiliently outwardly such that the alignment guide may pass readily over the femoral head. As the alignment guide moves into position, the leaf spring will return to the rest position. The alignment guide can therefore be regarded as being a snap-fit on the femoral head. This enables the alignment guide to be readily fitted and removed without the need for the surgeon to operate complex mechanical arrangements. The alignment guide can also be readily removed as when sufficient force is applied in a direction away from the femoral neck, the or each cantilever spring will move outwardly to enable the alignment guide to be removed from the femoral head.

In an alternative arrangement, additional flexibility to assist the positioning of the alignment guide on the femoral head may be provided in the support member. In one arrangement, the support member may comprise two segments, connected at the upper end and movable such that in use the segments can be moved from a first open position to a second closed position. The two segments may be produced as a single part, the resilience of which enables the two segments to move from the open to the closed position. In an alternative arrangement, the two segments are separate and are connected by any suitable means. For example, the two components may be hinged together to facilitate the movement from the first open position to the second closed position. In this arrangement, each segment may include an annular locking member extending from the segment inwardly towards the other segment. Once the alignment guide is in the desired position, the cannulated rod can be inserted through the annular locking members to lock the alignment guide in position.

The location means at the distal end of the locator arm may be of any suitable configuration. In one arrangement, the location means may simply be the end of the arm rather than being a separate arrangement. However, in another arrangement a toothed head or other arrangement may be used. This may be formed integrally with the locator arm or may be formed separately and then connected to the locator arm by any suitable means. In a still further alternative arrangement, the distal end of the locator arm may be shaped such that an elbow in the arm is formed which in use engages with the joint between the femoral head and the femoral neck.

The locator arm comprising the cantilever spring may be formed completely of material which will act as a cantilever spring or in one arrangement, only a portion, generally the portion nearest the distal end of the locator arm will be comprised of the cantilever spring.

The locator arms may be arranged such that in use they are at equally spaced circumferential locations around the femoral head. Alternatively they may be unequally spaced. In one arrangement, where four arms are present they may be spaced such that an angle between a first and second adjacent arms is acute and said third and fourth arm is acute while that between first and fourth arms and second and third arms is obtuse.

The locator arms may be connected to the support member by any suitable means. In one arrangement, the arms may be hinged to the support member. In one embodiment the locator arms may be formed as a single integral component. In a second embodiment two arms may be formed as a single integral component. For example, where there are four arms present, opposite arms may be formed as a single component.

The locator arms may be of any suitable material. Suitable materials include those having sufficient resilience to act as a cantilever spring.

In use, a stabilising ring may be used to extend around the locator arms.

The or each locator arm may be shaped along its length such that in use the distal ends will sit in the correct position on the femoral head. In one arrangement the or each arm will curve for at least a portion of it's length inwardly of the alignment guide.

The or each locator arm may be permanently connected to the support member or integrally manufactured therewith. In one alternative arrangement the or each locator arm may be demountable from the support member. The entire arm may be demountable or at least a portion may be demountable therefrom. Any suitable arrangement for connecting the demountable arm to the support member may be used. In are arrangement a slot may be provided in the demountable portion which may interact with a co-operating protrusion on the support member. The protrusion may be provided with a lip an its upper level such that once the arm is in position, it can be moved so that a portion of the arm sits under the lip to lock the arm in position. In once arrangement, the means for connecting the arm to the support member may be similar to that conventianally used to attach a scalpel blade to a scalpel handle.

Using demountable arms enables the surgeon to use single-use arms or arms sized for different sized femoral heads.

As will be discussed in more detail below, at least one arm may include other features to enable the surgeon to verify the positioning of the alignment guide and to facilitate the various operation steps which have to be taken. Whilst these can be located on one or more of the locator arms, and some may be located on one arm and some on the other, where present, for simplicity of manufacture and use, these additionally features may be provided on one arm.

As replacement femoral heads are available in a variety of sizes, a range of alignment guides may be provided wherein the locator arms on a particular size of guide, correspond to a particular size of replacement head.

The support member may be of any suitable configuration which will enable the locator arms to be held in position and which will have a cannulated rod to enable the surgeon to carry out the drilling step.

The cannulated rod may be adjustable with respect to the support arrangement. In one arrangement the rod is a sliding fit in the support. Once in the desired position the cannulated rod will preferably be lockable such that once locked further movement is prevented. Any suitable locking means may be used with a locking screw being preferred. In an alternative arrangement the rod may be fixed to the support member or be integral therewith.

The cannulated rod will in use enable the surgeon to position the guide wire. The cannulated rod may have a slot extending along at least a part of the length of the rod to assist in removing the tool from the guide wire once it is in position.

Teeth may be provided at the distal end thereof which can be driven into the surface of the femoral head. When driven into the head, these teeth help to clamp the alignment tool in position and further stabilise it.

The cannulated rod may additionally function as a measuring or gauging device and thus the surface of the rod may include measuring indicia to assist the surgeon to know how deep they have cut.

It is important for the correct operation of the hip prosthesis and the well-being of the patient that the prosthesis is correctly sited. As all of the machining of the femoral head is taken from the position of the guide wire inserted into the head, it is imperative that this is inserted as correctly as possible. The correct axis for insertion of the guide wire into the head of the femur is approximately 30 degrees from the sagittal plane axis of the femur and 20 degrees in anteversion to allow for natural offset in each position.

The alignment guide of the present invention preferably includes means to provide the surgeon with a visual indication of the varus/valgus angle. In one arrangement, this may simply be a varus flag extending from the alignment guide to enable the surgeon to visually confirm that the alignment guide is in the desired position.

However, in a preferred arrangement, a goniometer may be included. This may be integral with the alignment guide or in a preferred arrangement may be connectable to the alignment guide. In use the goniometer will point directly at the centre of the knee and provide the correct angle for the stem on the resurfacing head. The goniometer may be connectable to the alignment guide by any suitable means. In one arrangement, interlocking means may be provided on the support member, to engage corresponding features on the goniometer. Suitable indicating means, include those having a Y-shaped fork the tines of which will engage with a cooperating portion of the support member.

In an alternative arrangement, the goniometer may be a simple rod having an upper portion and a lower portion angled to the upper portion by an appropriate angle which may be 40°C. In use the distal end of the upper portion may be connected to the alignment guide by any suitable means. In one arrangement, the end of the upper portion may be inserted into a cooperating slot in a locating arm. For ease of use, each locating arm may be provided with a slot to accept the goniometer. This arrangement means that in use, the surgeon can select the most convenient support arm.

In order to indicate the correct anteversion angle, the alignment guide may additionally include anteversion indicating means. Whilst this may be integral with the guide and may be an anteversion flag extending from the alignment guide, in one arrangement, it may be separate therefrom and demountable from the alignment guide.

In an alternative arrangement, the anteversion indicating means may include a biting element such that the indicating means will connect with the femoral neck and also take a varus/valgus angle therefrom. The biting element may be of any suitable configuration. In one arrangement it may be a toothed block. The block may have a concave face between the teeth. The block may comprise four teeth, the teeth will preferably be configured and spaced on the block such that in use they interact with the inferior part of the neck of the femur to cause the tool to be angled at the optimum position. Thus the teeth will enable the tool to be clamped at the correct anteversion angle and at the correct angle from the sagittal plane with these angles being fixed by the femur itself. It is generally believed that there is a portion of the inferior femoral neck located from the head/neck junction of the femur to a position about 2 cms from the head/neck junction which is parallel to the optimum angle for the positioning of the stem of the prosthesis and hence this is often used as an alignment reference.

The optimum position of the tool may be achieved with four teeth in a generally square configuration. The teeth are preferably spaced at from about 10 to about 25 mm apart. They are most preferably spaced at about 15 mm.

According to a second aspect of the present invention there is provided a kit comprising at least one alignment guide in accordance with the above first aspect, and a goniometer.

The alignment guide of the present invention may be used in a method of preparing the head of a femur for femoral head resurfacing wherein the method comprises:
exposing the head of a femur;
locating the alignment guide according to the above first aspect on the head of the femur; and
machining the head of the femur.

To use the alignment guide most successfully an assessment of the head size should be made prior to machining and use of the guide in order to select the appropriate sized alignment guide. The correctly selected guide may provide a visual indication as to whether the correct. However, a more accurate method would be to measure the head size using, for example, calipers.

During the surgery, a well may be drilled into the head of the femur via the cannulated rod. This well may be the definite hole diameter required of approximately 8 mm and drilled to a depth determined by the tube touching the head. A check may be made with a stylus once the tool is removed and cylinder cutters used guided over a peg placed in the well.

These cutters are arranged such that the diameter cut will be correct for the head size chosen and will bottom on the top of the cut head such that the teeth of the cutter do not dangerously over-sail the head-neck junction and cause soft tissue damage or neck notching.

Thus the method preferably comprises:
exposing the head of the femur;
locating the alignment guide according to the above first aspect on the head of the femur;
inserting a drill and drilling a well into the head of the femur;
removing the drill;
removing the alignment guide;
removing the top of the head of the femur;
inserting a guide rod into the well;
locating a sleeve cutter on the guide rod and cutting the head; and
optionally chamfer cutting the head.

The correct axis for insertion of the guide wire into the head of the femur is approximately 30 degrees from the sagittal plane axis of the femur and in anteversion to allow for the natural offset in each position. Thus the tool of the present invention is configured such that in use the cannulated bore will be located such that the guide wire or drill is inserted at the correct angle. The arrangement of the present invention allows the surgeon to place, and to visually check that the tool is in the correct orientation, and position centered on the femoral head-neck junction.

It will be understood that whilst the tool of the present invention offers particular advantages for minimal invasive surgery, it can also be used in conventional surgical techniques.

The tool of the present invention may be used with all sizes of resurfacing head.

The present invention will now be described by way of example with reference to the accompanying figures in which:
- Figure 1: is a side view of the alignment guide of one embodiment of the present invention;
- Figure 2: is a perspective view of the arrangement of Figure 1;
- Figure 3: is an exploded view of the alignment guide showing the components;
- Figure 4: is a side view of the arrangement of Figure 3;
- Figure 5: is a side view of the alignment guide including connection means for the goniometer;
- Figure 6: is a perspective view of the arrangement of Figure 5;
- Figure 7: is a perspective view of the alignment guide of Figure 5 with the connection means for the goniometer removed;
- Figure 8: is a perspective view of the alignment guide in position on the femoral head with the goniometer in position and the guidewire inserted;
- Figure 9: is the arrangement of Figure 8 viewed from the side;
- Figure 10: is a view from beneath of the arrangement of Figure 9;
- Figure 11: is a cross-section of an alternative arrangement of the present invention in position on a femoral head; with the cannulated rod in position;
- Figure 12: is a schematic illustration of the arrangement illustrated in Figure 11 representing the operation of the embodiment;
- Figure 13: is a cross-section through the support member and support arms of the arrangement illustrated in Figure 11;
- Figure 14: is a partial view from above of the arrangement of Figure 11 illustrating the locating means for the goniometer;
- Figure 15: is an illustration of a goniometer for use in the alignment guide of the second embodiment;
- Figure 16: is a perspective view of the alignment guide as illustrated in Figure 11 from above;
- Figure 17: is a perspective view of the alignment guide as illustrated in Figure 11 from the side;
- Figure 18: is a perspective view of the alignment guide as illustrated in Figure 11 from below;
- Figure 19: is a perspective view from below of a second alternative arrangement of the present invention is an open configuration;
- Figure 20: is a perspective view from above of the embodiment of Figure 19;
- Figure 21: is a side view of the second alternative arrangement;
- Figure 22: is a view from below of the second alternative arrangement;
- Figure 23: is a perspective side view from below of the second alternative arrangement; and
- Figure 24: is a perspective side view from above of the second alternative arrangement.

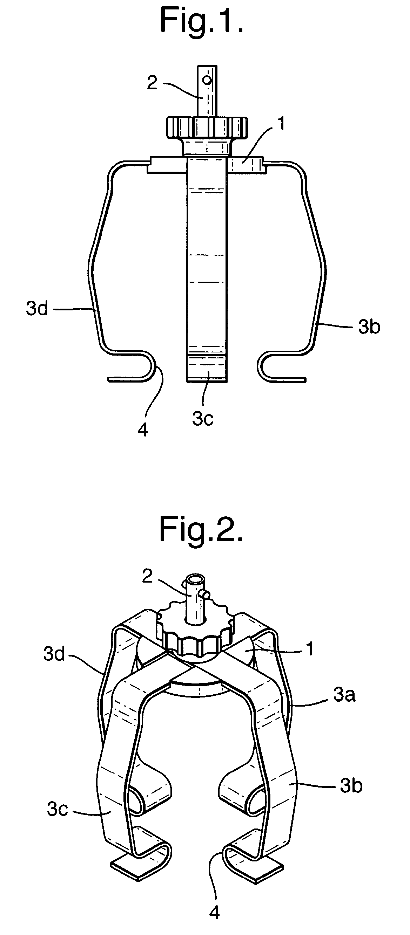

As illustrated in Figures 1 and 2, the alignment guide of the present invention comprises a support member 1 including a cannulated rod 2. Four locator arm 3a, b, c and d extend from the support member. Each locator arm has a locator member 4 located at its distal end. The locator member 4 comprises an elbow formed in the locator arm.

Each locator arm 3a, b, c and d is a cantilever spring connected to the cannulated rod.

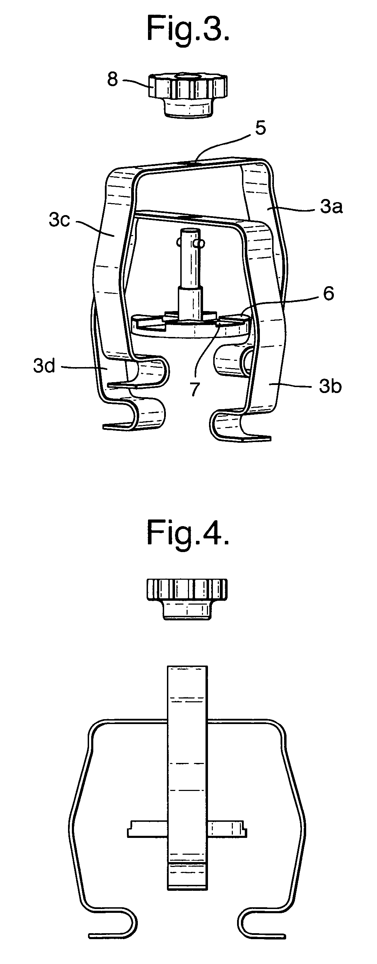

As illustrated in Figures 3 and 4, opposing arms, 3a - 3c and 3b - 3d may be formed from a single component which has an aperture 5 provided therein through which the cannulated rod 2 can pass.

In this arrangement, the support member is shaped to receive the arms across its upper surface 6. For example, channels 7 may be provided in which the arms can lie.

A locking means 8 such as a knurled knob may be provided to lock the locator arms to the support member 1.

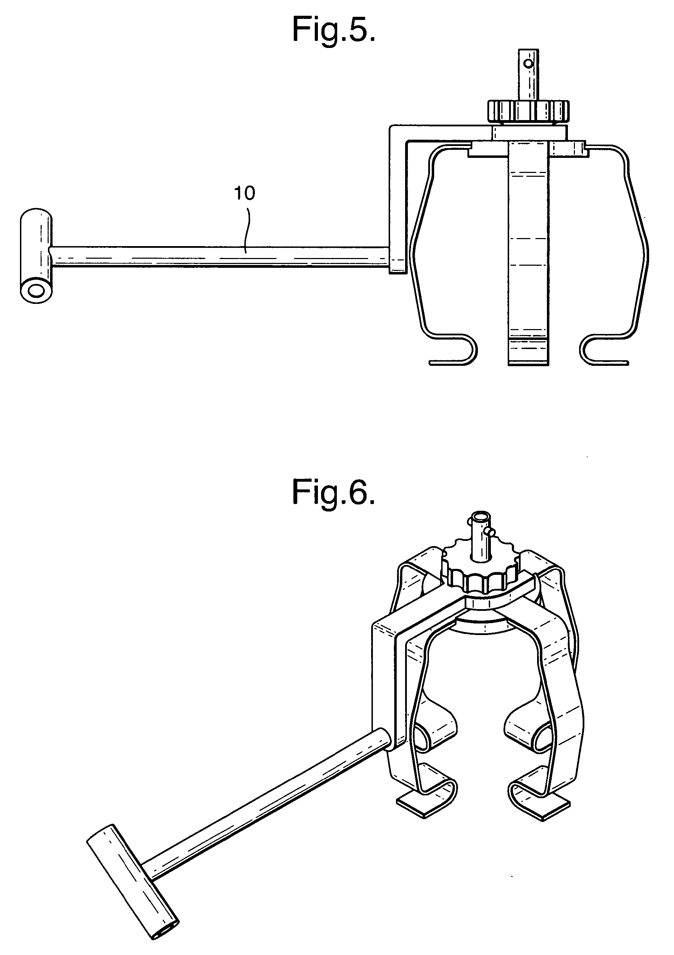

The goniometer support member 10 and its location on the support member is illustrated in Figures 5 and 6.

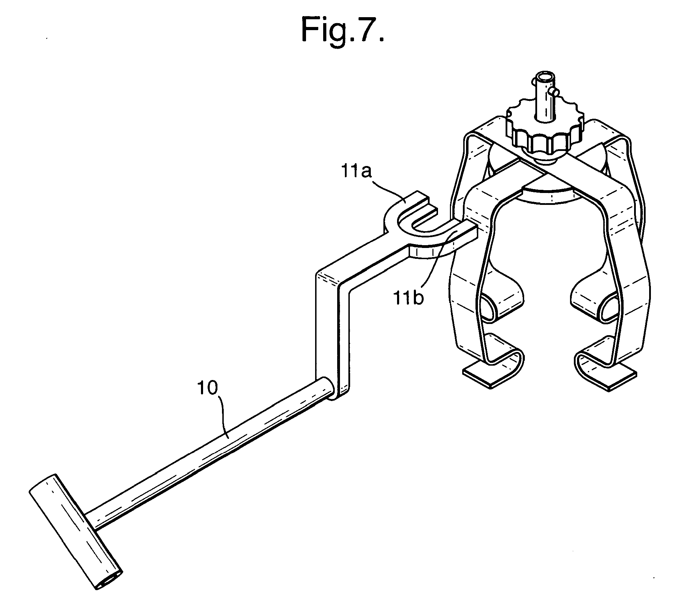

As illustrated in Figure 7 the goniometer support member 10 may have, at the end which As illustrated in Figure 7 the goniometer support member 10 may have, at the end which is to be connected to the support member, a fork arrangement having two tines 11 a and 11 b which when the goniometer support member is connected to the support member sit on the support member and extend around the cannulated rod.

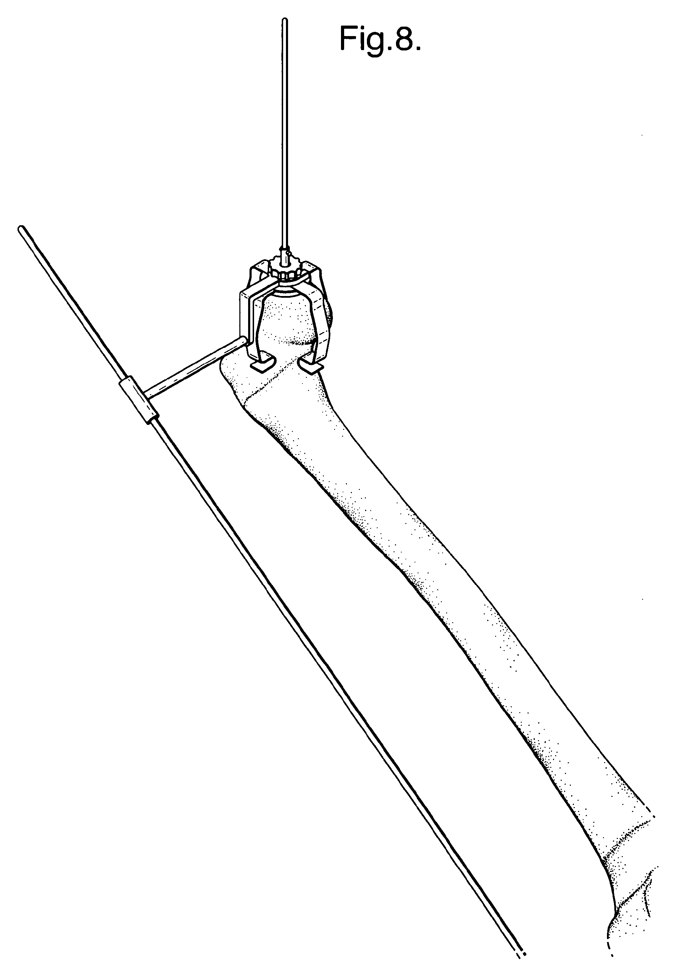

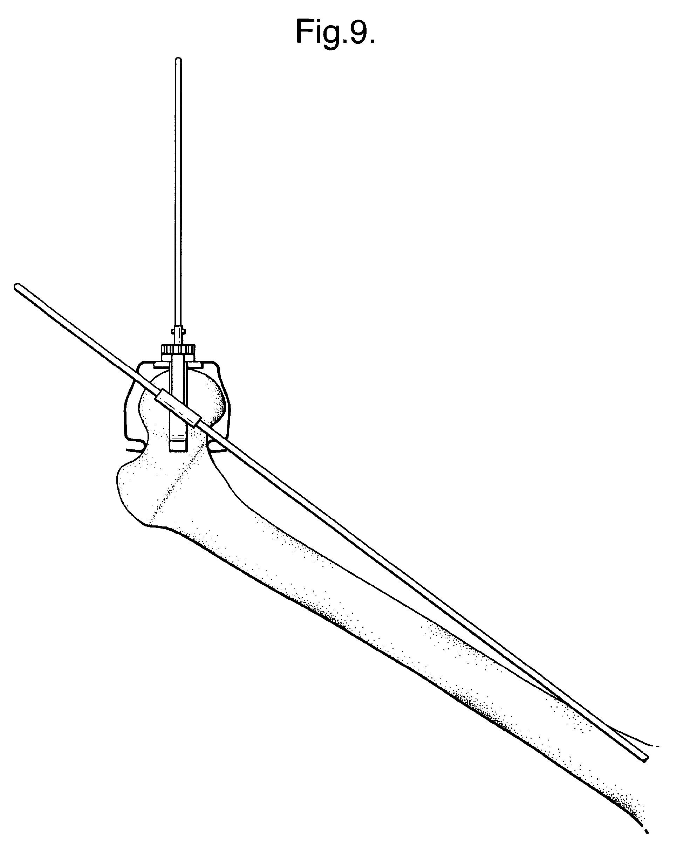

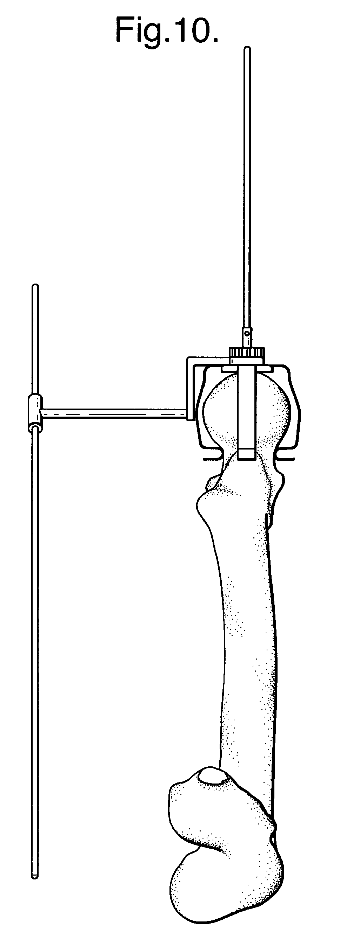

In use, once the surgeon has assessed the size of the femoral head, preferably using calipers, and the appropriate sized locator means has been selected, the alignment guide of the present invention is placed around the femoral head as illustrated in Figures 8, 9 and 10. The goniometer means is then connected. The rod 30 of the goniometer should be pointed at the knee. Adjustments of the alignment guide can be undertaken until the orientation has been fine-tuned.

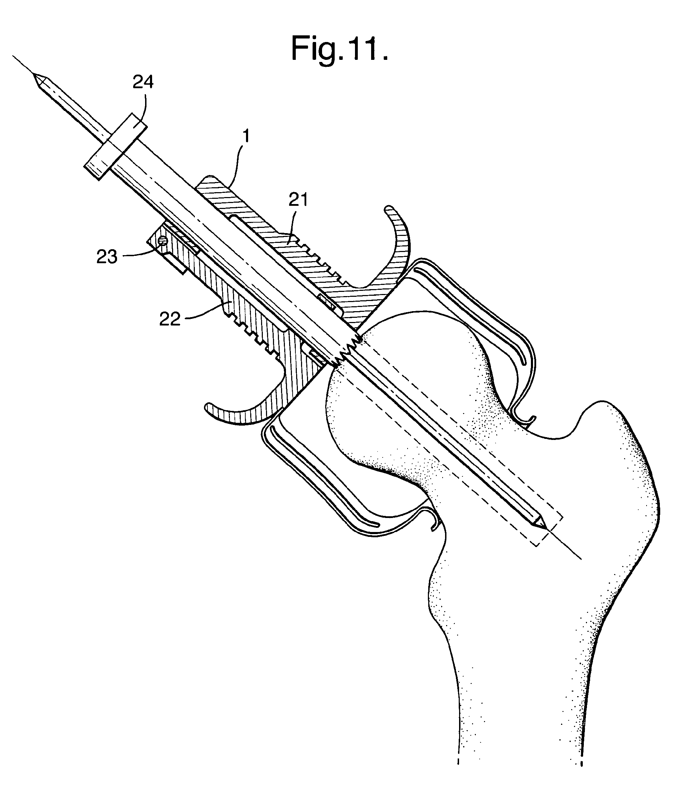

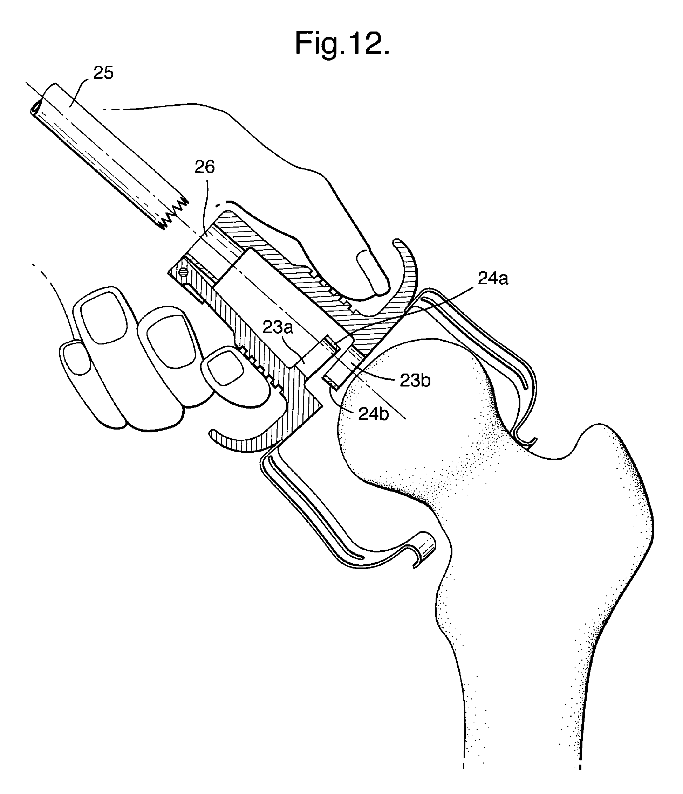

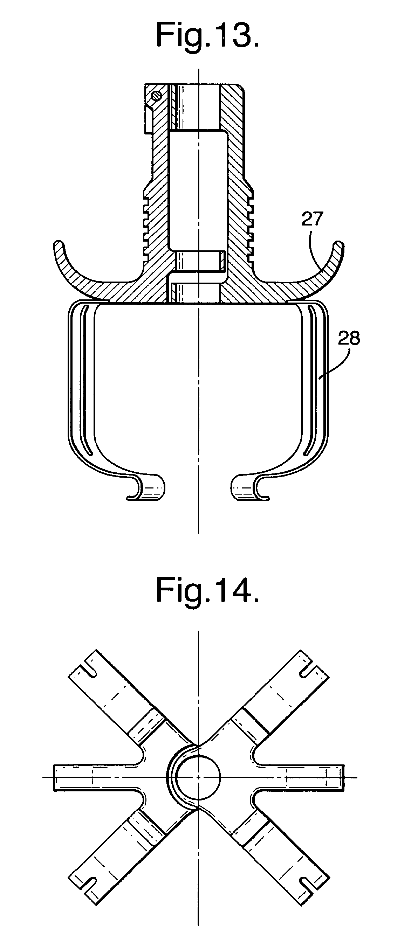

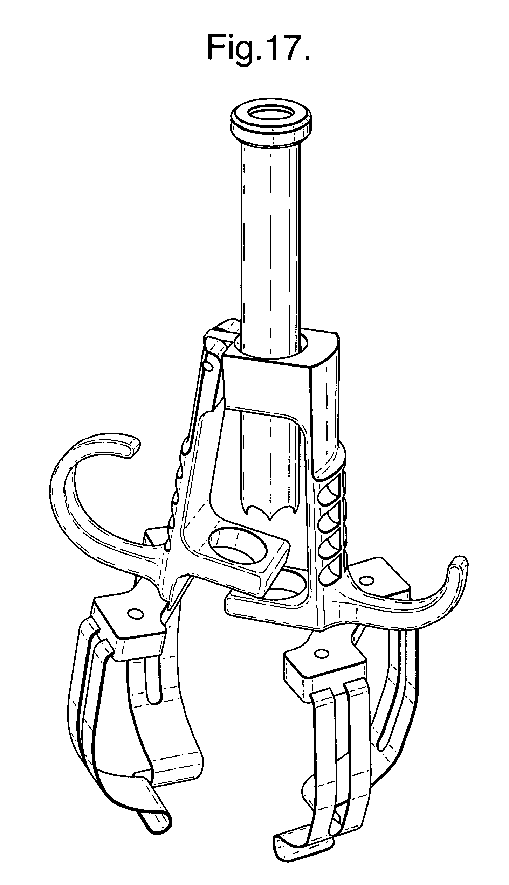

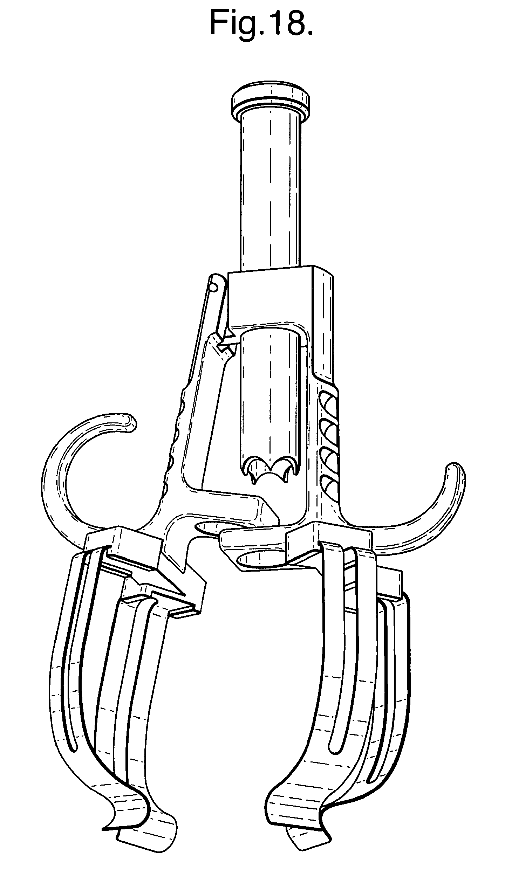

Figures 11 to 18 illustrate an alternative arrangement of the alignment guide of the present invention. In this alternative arrangement, support member 1 comprises two segments 21 1 and 22 connected together by a hinge 23. As illustrated in Figure 12, the two segments 21 1 and 22 move apart to enable the alignment guide to be located in position on the femoral head. Once the locator arms surround the head, the user moves the segments toward one another such that apertures 23a and 23b in the two annular locking means 24a and 24b. Once the apertures in the annular locking means are aligned, the cannulated rod 25 can be inserted through an aperture 26 in the top end of the support member and then through the two apertures in the two annular locking means 24a and 24b such that the support members segments and the locking arms are locked in the desired position.

Finger guards 27 may be present. Slots 28 into which a goniometer may be inserted is provided in each locator arm.

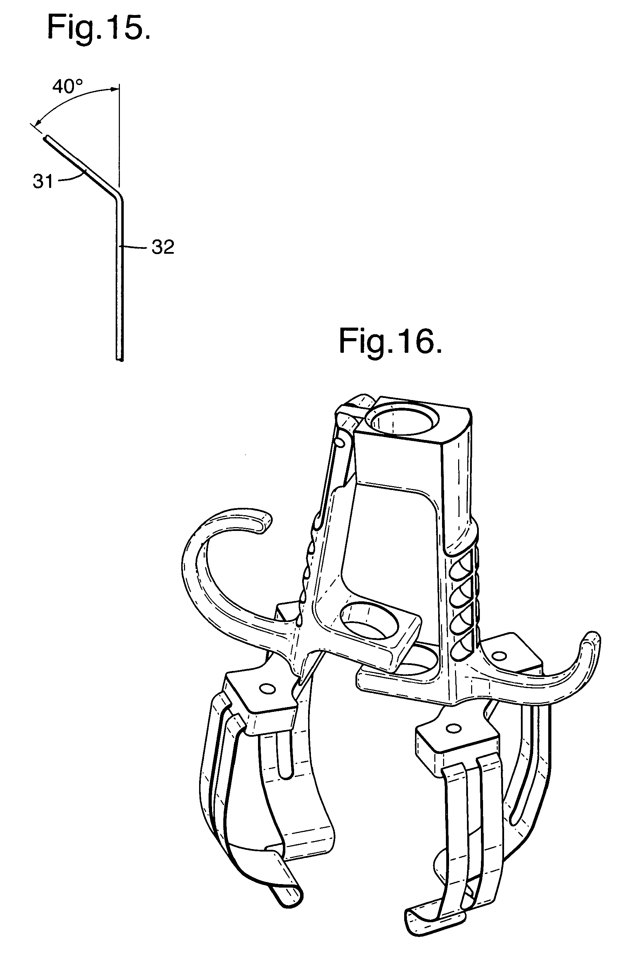

One example of a suitable goniometer is illustrated in Figure 15. Thus goniometer is formed from a single rod having an upper region 31 and a lower region 32. The upper region being angled to the plane of the lower region by 40° In use the end of the upper region can be located in one of the slots 28 and the lower region can be pointed at the knee to check the alignment of the alignment guide.

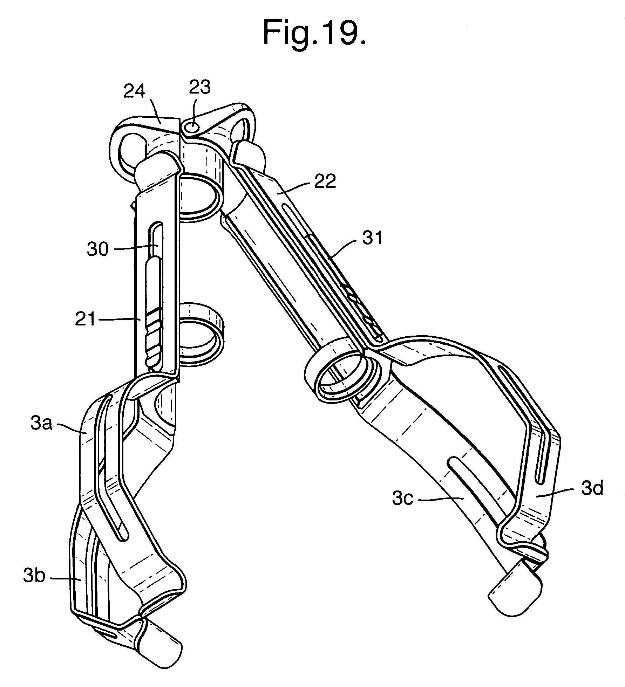

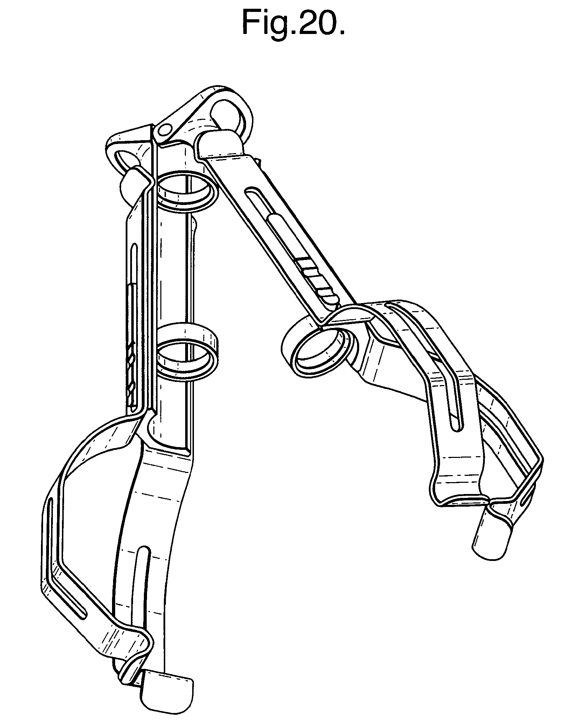

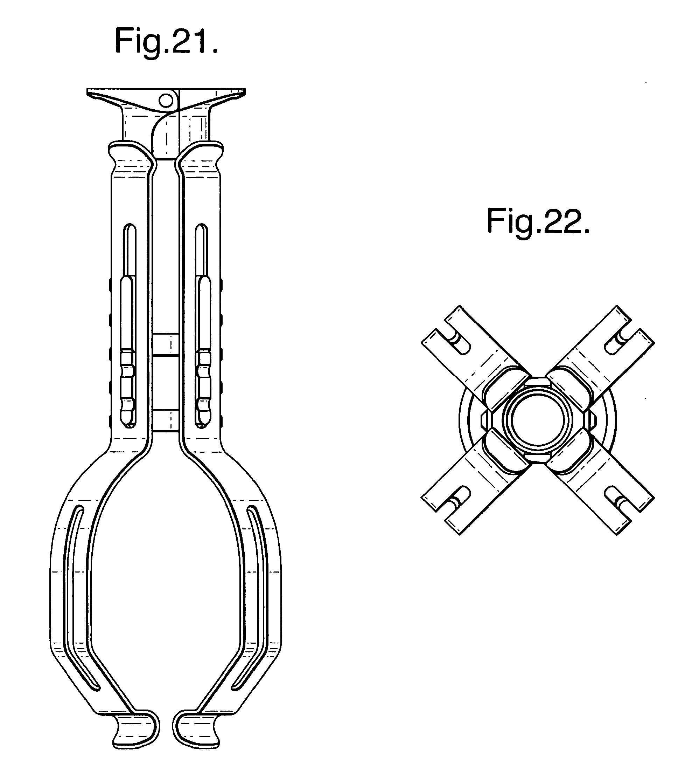

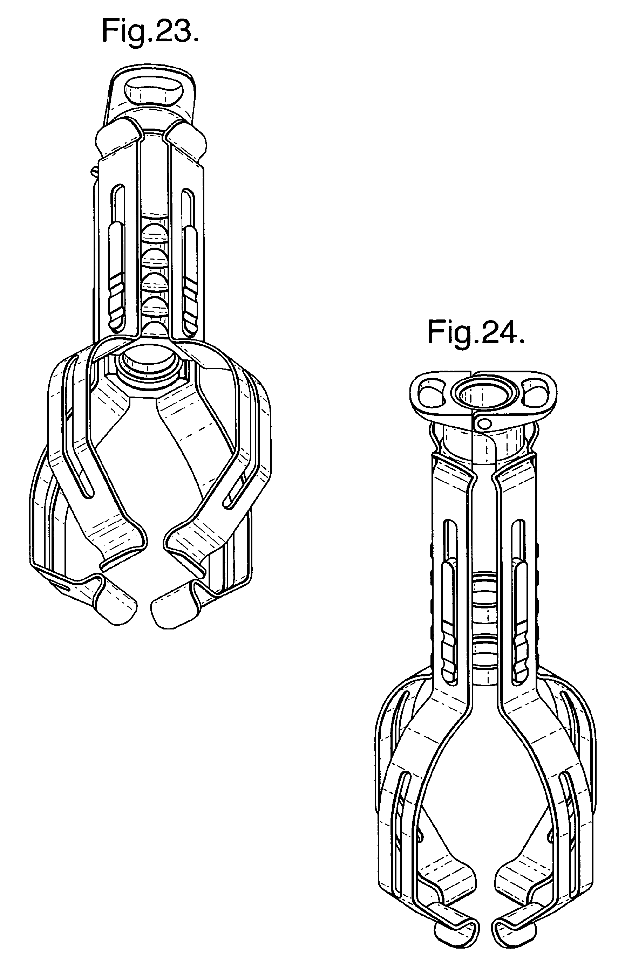

An alternative arrangement is illustrated in Figures 19 to 24. This arrangement is similar to that of Figures 11 to 18. The locator arms, 3a, 3b, 3c and 3d, are demountable from the support member. Each arm includes a slot 30 which enables the arms to be a snap-fit on protrusions 31 on the support member.

## Claims

1. An alignment guide for use in femoral head surgery comprising:
a support member comprising a cannulated rod;
at least three locator arms at least a portion of at least one of which comprises a cantilever spring, each of said arms having a proximal end connected to the support member and a distal end having location means for location at the femoral head/neck junction,
said at least three locator arms being spaced such that in use they extend around the femoral head and prevent relative movement between the alignment guide and the femoral head.

2. An alignment guide according to Claim 1 wherein each locator arm comprises a cantilever spring.

3. An alignment guide according to Claim 1 or 2 wherein the alignment guide includes four locator arms each of which comprises a cantilever spring.

4. An alignment guide according to any one of Claims 1 to 3 wherein the locator means are formed by the locator arm being shaped such that an elbow in the arm is formed which in use engages with the joint between the femoral head and the femoral neck.

5. An alignment guide according to any one of Claims 1 to 4 wherein the locator arms are at equally spaced circumferential locations.

6. An alignment guide according to any one of Claims 1 to 4 wherein the locator arms are unequally spaced.

7. An alignment guide according to Claim 6 wherein there are four locator arms and said arms are spaced such that an angle between a first and second adjacent arms is acute and the angle between said third and fourth arm is acute while that between first and fourth and second and third is obtuse.

8. An alignment guide according to any one of Claims 1 to 7 wherein two arms are formed as a single integral component.

9. An alignment guide according to any one of Claims 1 to 7 wherein a stabilising ring is used to extend around the locator arms.

10. An alignment guide according to any one of Claims 1 to 7 wherein the support member is formed from two segments movable from a first open position to a second open position.

11. An alignment guide according to Claim 10 wherein the two segments are hinged together.

12. An alignment guide according to Claim 10 wherein the two segments are formed as a single piece.

13. An alignment guide according to any one of Claims 10 to 12 wherein each segment includes an annular locking means.

14. An alignment guide according to Claim 13 wherein, in use, the cannulated rod passes through the annular locking means in each segment to lock the alignment guide in position.

15. An alignment guide according to any one of Claims 1 to 14 wherein the or each locator arm is shaped along its length such that in use the distal ends will sit in the correct position on the femoral head.

16. An alignment guide according to any one of Claims 1 to 15 wherein the or each locator arm is demountable.

17. An alignment guide according to any one of Claims 1 to 16 wherein the alignment guide includes means to provide the surgeon with a visual indication of the varus/valgus angle.

18. An alignment guide according to Claim 17 wherein a varus flag extends from the alignment guide.

19. An alignment guide according to Claim 18 wherein at least one locator arm includes a slot into which in use the goniometer may be inserted.

20. An alignment guide according to any one of Claims 17 to 19 additionally including a goniometer.

21. An alignment guide according to Claim 20 wherein the goniometer is connectable to the support member by means of a Y-shaped fork the tines of which engage with a cooperating portion of the support member.

22. An alignment guide according to Claim 20 wherein the goniometer comprises a rod having an upper portion and a lower portion, said upper portion being angled to the lower portion at an angle of 40°.

23. A kit comprising at least one alignment guide according to any one of Claims 1 to 22 and a goniometer.
